# EUROPEAN PATENT APPLICATION

(11) **EP 1 369 483 A1**
(43) Date of publication of application: **10.12.2003**
(21) Application number: 02012302.2
(22) Date of filing: 04.06.2002
(51) Int. Cl.: C12N 15/29, C07K 14/415, C07K 16/16, A61K 39/36

(54) **Allergen from mugwort pollen**

(71) Applicant: BIOMAY Produktions- und Handels- Aktiengesellschaft, 1030 Wien (AT)
(72) Inventor: Ferreira, Fatima, 5020 Salzburg (AT); Hubinger, Gudrun, 5020 Salzburg (AT); Ebner, Christof, 2345 Brunn am Gebirge (AT); Richter, Klaus, 5081 Anif (AT)
(74) Representative: Keller, Günter, Dr.

(57) **Abstract**

The present invention relates to a 40.9 kDa allergen from mugwort pollen, to polypeptides derived therefrom and polynucleotides encoding the same. The invention further pertains to antibodies directed against the allergen and to the use of the materials described for the manufacture of a medicament for the treatment or prevention of an allergic disorder.

SEQUENCE LISTING

<110> Biomay Produktions- und Handels-Aktiengesellschaft

<120> Allergenic protein from mugwort pollen

<130> Allergenic protein from mugwort pollen

<140>
<141>

<160> 9

<170> PatentIn Ver. 2.1

<210> 1
<211> 396
<212> PRT
<213> Artemisia vulgaris

<400> 1
Met Glu Lys His Tyr Phe Val Ile Leu Phe Thr Ala Ala Phe Val Phe
 1               5                  10                  15

Val Gly Ala Ala Ala Arg Ala Asp Ile Gly Asp Glu Leu Glu Ala Ala
            20                  25                  30

Gln Phe Asn Ser Thr Arg Arg Gly Leu His Glu Cys Ala Ala His Asn
        35                  40                  45

Ile Ile Asp Lys Cys Trp Arg Cys Lys Ala Asp Trp Glu Lys Asn Arg
    50                  55                  60

Gln Ala Leu Ala Lys Cys Ala Gln Gly Phe Ala Lys Gly Thr Thr Gly
65                  70                  75                  80

Gly Leu Gly Gly Glu Ile Tyr Val Val Thr Asp Cys Ser Asp Asp Asn
                85                  90                  95

Ala Ala Asn Pro Lys Pro Gly Thr Leu Arg Cys Gly Val Thr Gln Asp
            100                 105                 110

Lys Pro Leu Trp Ile Ile Phe Lys Lys Asp Met Val Ile Lys Leu Lys
        115                 120                 125

His Glu Leu Val Ile Asn Lys Asp Lys Thr Ile Asp Gly Arg Gly Ala
    130                 135                 140

Asn Val Glu Ile Thr Cys Gly Gly Leu Thr Ile His Asn Val Cys Asn
145                 150                 155                 160

Val Ile Ile His Asn Ile His Ile His Asp Ile Lys Val Thr Glu Gly
                165                 170                 175

Gly Ile Ile Lys Ala Thr Asp Ala Lys Pro Gly His Arg His Lys Ser
            180                 185                 190

Asp Gly Asp Gly Ile Cys Val Ala Gly Ser Ser Lys Ile Trp Ile Asp
        195                 200                 205

His Cys Thr Leu Ser His Gly Pro Asp Gly Leu Ile Asp Val Thr Leu
    210                 215                 220

Gly Ser Thr Ala Val Thr Ile Ser Asn Cys Lys Phe Ser His His Gln
225                 230                 235                 240

Lys Ile Leu Leu Leu Gly Ala Asp Asn Ser His Val Asp Asp Lys Lys
                245                 250                 255

Met His Val Thr Val Ala Phe Asn Arg Phe Ala Glu Ala Cys Asp Gln
            260                 265                 270

Arg Met Pro Arg Cys Arg Phe Gly Phe Phe Gln Val Val Asn Asn Asp
        275                 280                 285

Tyr Thr Ser Trp Gly Thr Tyr Ala Ile Gly Gly Ser Ala Asn Pro Thr
    290                 295                 300

Ile Leu Ser Gln Gly Asn Arg Phe His Ala Pro Asn Asp Pro Met Lys
305                 310                 315                 320

Lys Asn Val Leu Val Arg Ala Asp Ala Pro His Thr Glu Ser Met Lys
                325                 330                 335

Trp Asn Trp Arg Ser Glu Lys Asp Leu Leu Glu Asn Gly Ala Ile Phe
            340                 345                 350

Val Ala Ser Gly Cys Asp Pro His Leu Thr Pro Glu Gln Lys Ser His
        355                 360                 365

Leu Ile Pro Ala Glu Pro Gly Ser Ala Val Leu Gln Leu Thr Ser Cys
    370                 375                 380

Ala Gly Thr Leu Lys Cys Val Pro Gly Lys Pro Cys
385                 390                 395

<210> 2
<211> 1188
<212> DNA
<213> Artemisia vulgaris

<400> 2
atggaaaaac attattttgt tatattgttc accgcagcgt ttgttttcgt gggtgcagct 60
gctcgggctg acattggtga tgagctcgaa gcggctcaat ttaattcaac aaggaggggc 120
ttacacgaat gtgcagcaca taacataata gacaagtgtt ggaggtgcaa agctgattgg 180
gaaaaaaacc gacaagcatt agccaaatgc gcgcaaggtt ttgcaaaggg aacaactggc 240
ggattgggag gggaaatcta cgtggtgact gattgttcag atgacaatgc tgcaaatcca 300
aagccaggga cacttcgttg tggtgtcacc caagataaac ctttgtggat catctttaag 360
aaagatatgg tcataaaact taaacacgag cttgtgataa acaaagacaa gacaattgat 420
ggaagaggtg caaatgttga gatcacttgt ggcggtctca ccattcacaa cgtttgcaat 480
gtgatcattc ataacattca catacatgat attaaagtaa ccgaaggtgg aattattaag 540
gcaacggacg ctaaaccagg acatagacat aagagcgacg gagatggtat ttgtgttgct 600
ggttcttcaa agatatggat cgatcattgc acacttagtc atggtccaga tggccttatt 660
gatgtcacgt tgggtagcac agccgttacc atttccaatt gcaaatttag ccatcaccaa 720
aaaattctat tactcggagc agacaattca catgtagacg ataaaaaaat gcatgtcaca 780
gtagcattca acaggttcgc agaagcatgt gatcaaagaa tgccacgatg tcgatttgga 840
tttttccaag ttgttaacaa tgactacacc agctggggaa cgtacgccat tggtggtagt 900
gccaatccta ctatccttag ccaaggcaac cgattccatg ctccgaatga cccaatgaag 960
aaaaatgtgt tggtgagggc tgatgcacca catacagagt caatgaagtg gaattggaga 1020
tctgagaaag acttgttaga aaatggagct atatttgtag catcagggtg cgacccgcat 1080
ctaaccccgg aacaaaaaag ccatttgatt ccagctgaac caggatcagc agttcttcaa 1140
ctcaccagtt gtgctggcac gctcaaatgc gttcctggaa aaccttgt 1188

<210> 3
<211> 1188
<212> DNA
<213> Artemisia vulgaris

<400> 3
atggaaaaac attattttgt tatattgttc accgcagcgt ttgttttcgt gggtgcagct 60
gctcgggctg acattggtga tgagctcgaa gcggctcaat ttaattcaac aaggaggggc 120
ttacacgaat gtgcagcaca taacataata gacaagtgtt ggaggtgcaa agctgattgg 180
gaaaaaaacc gacaagcatt agccaaatgc gcgcaaggtt ttgcaaaggg aacaactggc 240
ggattgggag gggaaatcta cgtggtgact gattgttcag atgacaatgc tgcaaatcca 300
aagccaggga cacttcgttg tggtgtcacc caagataaac ctttgtggat catcttcaag 360
aaagatatgg tcataaaact taaacacgag cttgtgataa acaaagacaa gacaattgat 420
ggaagaggtg caaatgttga gatcacttgt ggcggtctca ccattcacaa cgtttgcaat 480
gtgatcattc ataacattca catacatgat attaaagtaa cggaaggtgg aattattaag 540
gcaacggacg ctaaaccagg gcatagacat aagagcgacg gagatggtat ttgtgttgct 600
ggttcttcga agatatggat cgatcattgc acacttagtc atggtccaga tggccttatt 660
gatgtcacgt tgggtagcac agccgttacc atttccaatt gcaaatttag ccatcaccaa 720
aaaattctat tactcggagc agacaattca catgtagacg ataaaaaaat gcatgtcaca 780

gtcgcattca acaggttcgc agaagcatgt gatcaaagaa tgccacgatg tcgatttgga 840
tttttccaag ttgttaacaa tgactacacc agctggggaa cgtacgccat tggtggtagc 900
gccaatccta ctatccttag ccaaggcaac cgattccatg ctcccaatga cccaatgaag 960
aaaaatgtgt tggtgagggc tgatgcacca catacagagt caatgaagtg gaattggaga 1020
tctgagaaag acttgttaga aaatggagct atatttgtag catcagggtg cgacccgcat 1080
ctaaccccgg aacaaaaaag ccatttaatt ccagctgaac caggatcagc agttcttcaa 1140
ctcaccagtt gtgctggcac gctcaaatgc gttcctggaa aaccttgt 1188

<210> 4
<211> 41
<212> DNA
<213> Artificial Sequence

<220>
<223> Description of Artificial Sequence: Primer

<400> 4
gagagagacc atggctcggg ctgacattgg tgatgagctc g 41

<210> 5
<211> 42
<212> DNA
<213> Artificial Sequence

<220>
<223> Description of Artificial Sequence: Primer

<400> 5
gagagagact cgagttaaca aggttttcca ggaacgcatt tg 42

<210> 6
<211> 1523
<212> DNA
<213> Artemisia vulgaris

<400> 6
ggaattcggc acgagaaatc tacaaaaatt gataaaaata aaaataacaa agcgagtcat 60
tggcttgcat acatggctac atgattcgct ttagacaaca caataaataa tcagcaatat 120
ataaagtacc ttcggtactt tgagatagaa agttgtaaaa aaagataatc atacaataca 180
atggaaaaac attattttgt tatattgttc accgcagcgt ttgttttcgt gggtgcagct 240
gctcgggctg acattggtga tgagctcgaa gcggctcaat ttaattcaac aaggaggggc 300
ttacacgaat gtgcagcaca taacataata gacaagtgtt ggaggtgcaa agctgattgg 360
gaaaaaaacc gacaagcatt agccaaatgc gcgcaaggtt ttgcaaaggg aacaactggc 420
ggattgggag gggaaatcta cgtggtgact gattgttcag atgacaatgc tgcaaatcca 480
aagccaggga cacttcgttg tggtgtcacc caagataaac ctttgtggat catctttaag 540

aaagatatgg tcataaaact taaacacgag cttgtgataa acaaagacaa gacaattgat 600
ggaagaggtg caaatgttga gatcacttgt ggcggtctca ccattcacaa cgtttgcaat 660
gtgatcattc ataacattca catacatgat attaaagtaa ccgaaggtgg aattattaag 720
gcaacggacg ctaaaccagg acatagacat aagagcgacg gagatggtat ttgtgttgct 780
ggttcttcaa agatatggat cgatcattgc acacttagtc atggtccaga tggccttatt 840
gatgtcacgt tgggtagcac agccgttacc atttccaatt gcaaatttag ccatcaccaa 900
aaaattctat tactcggagc agacaattca catgtagacg ataaaaaaat gcatgtcaca 960
gtagcattca acaggttcgc agaagcatgt gatcaaagaa tgccacgatg tcgatttgga 1020
tttttccaag ttgttaacaa tgactacacc agctggggaa cgtacgccat tggtggtagt 1080
gccaatccta ctatccttag ccaaggcaac cgattccatg ctccgaatga cccaatgaag 1140
aaaaatgtgt tggtgagggc tgatgcacca catacagagt caatgaagtg gaattggaga 1200
tctgagaaag acttgttaga aaatggagct atatttgtag catcagggtg cgacccgcat 1260
ctaaccccgg aacaaaaaag ccatttgatt ccagctgaac caggatcagc agttcttcaa 1320
ctcaccagtt gtgctggcac gctcaaatgc gttcctggaa aaccttgtta atagttatca 1380
cccacttttt ttttttattg ttattagcat tttttatact tgttaggatt gtagtggaat 1440
gagacattga tacgtgcatt acaagaccat tcatcaacat attttgctac tatcacatgt 1500
tcacgttaaa aaaaaaaaaa aaa 1523

<210> 7
<211> 1513
<212> DNA
<213> Artemisia vulgaris

<400> 7
ggaattcggc acgagaatct acaaaaattg ataaaaataa aaataaaaat aacaaagcga 60
gtcattggct tgcatacatg gctacatgat tcgctttaga caacacaata aataatcagc 120
aatatataaa gtaccttcgg tactttgaga tagaaagttg taaaaaaaga taatcataca 180
atacaatgga aaaacattat tttgttatat tgttcaccgc agcgtttgtt ttcgtgggtg 240
cagctgctcg ggctgacatt ggtgatgagc tcgaagcggc tcaatttaat tcaacaagga 300
ggggcttaca cgaatgtgca gcacataaca taatagacaa gtgttggagg tgcaaagctg 360
attgggaaaa aaaccgacaa gcattagcca aatgcgcgca aggttttgca aagggaacaa 420
ctggcggatt gggaggggaa atctacgtgg tgactgattg ttcagatgac aatgctgcaa 480
atccaaagcc agggacactt cgttgtggtg tcacccaaga taaacctttg tggatcatct 540
ttaagaaaga tatggtcata aaacttaaac acgagcttgt gataaacaaa gacaagacaa 600
ttgatggaag aggtgcaaat gttgagatca cttgtggcgg tctcaccatt cacaacgttt 660
gcaatgtgat cattcataac attcacatac atgatattaa agtaaccgaa ggtggaatta 720
ttaaggcaac ggacgctaaa ccaggacata gacataagag cgacggagat ggtatttgtg 780
ttgctggttc ttcaaagata tggatcgatc attgcacact tagtcatggt ccagatggcc 840
ttattgatgt cacgttgggt agcacagccg ttaccatttc caattgcaaa tttagccatc 900
accaaaaaat tctattactc ggagcagaca attcacatgt agacgataaa aaaatgcatg 960
tcacagtagc attcaacagg ttcgcagaag catgtgatca aagaatgcca cgatgtcgat 1020
ttggattttt ccaagttgtt aacaatgact acaccagctg gggaacgtac gccattggtg 1080
gtagtgccaa tcctactatc cttagccaag gcaaccgatt ccatgctccg aatgacccaa 1140
tgaagaaaaa tgtgttggtg agggctgatg caccacatac agagtcaatg aagtggaatt 1200
ggagatctga gaaagacttg ttagaaaatg gagctatatt tgtagcatca gggtgcgacc 1260
cgcatctaac cccggaacaa aaaagccatt tgattccagc tgaaccagga tcagcagttc 1320
ttcaactcac cagttgtgct ggcacgctca aatgcgttcc tggaaaacct tgttaatagt 1380

tatcacccac tttttatttt tattgttatt agcatttttt atacttgtta ggattgtagt 1440
ggaatgagac attgatacgt gcattacaag accattcatc aacatatttt gctaaaaaaa 1500
aaaaaaaaaa aaa 1513

<210> 8
<211> 1520
<212> DNA
<213> Artemisia vulgaris

<400> 8
gcggccgctc tagaactagt ggatcccccg ggctgcagga attcggcacg agaaattgat 60
aaaaataaaa ataaaaataa caaagcgagt cattggcttg catacatggc tacatgattc 120
gctttagaca acacaataaa taatcagcaa tatataaagt accttcggta ctttgagata 180
gaaagttgta aaaaaagata atcatacaat acaatggaaa aacattattt tgttatattg 240
ttcaccgcag cgtttgtttt cgtgggtgca gctgctcggg ctgacattgg tgatgagctc 300
gaagcggctc aatttaattc aacaaggagg ggcttacacg aatgtgcagc acataacata 360
atagacaagt gttggaggtg caaagctgat tgggaaaaaa accgacaagc attagccaaa 420
tgcgcgcaag gttttgcaaa gggaacaact ggcggattgg gaggggaaat ctacgtggtg 480
actgattgtt cagatgacaa tgctgcaaat ccaaagccag ggacacttcg ttgtggtgtc 540
acccaagata aacctttgtg gatcatcttc aagaaagata tggtcataaa acttaaacac 600
gagcttgtga taaacaaaga caagacaatt gatggaagag gtgcaaatgt tgagatcact 660
tgtggcggtc tcaccattca caacgtttgc aatgtgatca ttcataacat tcacatacat 720
gatattaaag taacggaagg tggaattatt aaggcaacgg acgctaaacc agggcataga 780
cataagagcg acggagatgg tatttgtgtt gctggttctt cgaagatatg gatcgatcat 840
tgcacactta gtcatggtcc agatggcctt attgatgtca cgttgggtag cacagccgtt 900
accatttcca attgcaaatt tagccatcac caaaaaattc tattactcgg agcagacaat 960
tcacatgtag acgataaaaa aatgcatgtc acagtcgcat tcaacaggtt cgcagaagca 1020
tgtgatcaaa gaatgccacg atgtcgattt ggatttttcc aagttgttaa caatgactac 1080
accagctggg gaacgtacgc cattggtggt agcgccaatc ctactatcct tagccaaggc 1140
aaccgattcc atgctcccaa tgacccaatg aagaaaaatg tgttggtgag ggctgatgca 1200
ccacatacag agtcaatgaa gtggaattgg agatctgaga aagacttgtt agaaaatgga 1260
gctatatttg tagcatcagg gtgcgacccg catctaaccc cggaacaaaa aagccattta 1320
attccagctg aaccaggatc agcagttctt caactcacca gttgtgctgg cacgctcaaa 1380
tgcgttcctg gaaaaccttg ttaatagtta tcacccactt tttattttta ttgttattag 1440
cattttttat acttgttagg attgtagtgg aatgagacat tgatacgtgc attacaagac 1500
caaaaaaaaa aaaaaaaaaa 1520

<210> 9
<211> 1535
<212> DNA
<213> Artemisia vulgaris

<400> 9
ggcggccgct ctagaactag tggatccccc gggctgcagg aattcggcac gagtgaaaat 60
ctacaaaaat tgataaaaat aaaaataaaa ataacaaagc gagtcattgg cttgcataca 120
tggctacatg attcgcttta gacaacacaa taaataatca gcaatatata aagtaccttc 180

ggtactttga gatagaaagt tgtaaaaaaa gataatcata caatacaatg gaaaaacatt 240
attttgttat attgttcacc gcagcgtttg ttttcgtggg tgcagctgct cgggctgaca 300
ttggtgatga gctcgaagcg gctcaattta attcaacaag gaggggctta cacgaatgtg 360
cagcacataa cataatagac aagtgttgga ggtgcaaagc tgattgggaa aaaaaccgac 420
aagcattagc caaatgcgcg caaggttttg caaagggaac aactggcgga ttgggagggg 480
aaatctacgt ggtgactgat tgttcagatg acaatgctgc aaatccaaag ccagggacac 540
ttcgttgtgg tgtcacccaa gataaacctt tgtggatcat ctttaagaaa gatatggtca 600
taaaacttaa acacgagctt gtgataaaca aagacaagac aattgatgga agaggtgcaa 660
atgttgagat cacttgtggc ggtctcacca ttcacaacgt ttgcaatgtg atcattcata 720
acattcacat acatgatatt aaagtaaccg aaggtggaat tattaaggca acggacgcta 780
aaccaggaca tagacataag agcgacggag atggtatttg tgttgctggt tcttcaaaga 840
tatggatcga tcattgcaca cttagtcatg gtccagatgg ccttattgat gtcacgttgg 900
gtagcacagc cgttaccatt tccaattgca aatttagcca tcaccaaaaa attctattac 960
tcggagcaga caattcacat gtagacgata aaaaaatgca tgtcacagta gcattcaaca 1020
ggttcgcaga agcatgtgat caaagaatgc cacgatgtcg atttggattt ttccaagttg 1080
ttaacaatga ctacaccagc tggggaacgt acgccattgg tggtagtgcc aatcctacta 1140
tccttagcca aggcaaccga ttccatgctc cgaatgaccc aatgaagaaa aatgtgttgg 1200
tgagggctga tgcaccacat acagagtcaa tgaagtggaa ttggagatct gagaaagact 1260
tgttagaaaa tggagctata tttgtagcat cagggtgcga cccgcatcta accccggaac 1320
aaaaaagcca tttgattcca gctgaaccag gatcagcagt tcttcaactc accagttgtg 1380
ctggcacgct caaatgcgtt cctggaaaac cttgttaata gttatcaccc actttttatt 1440
tttattgtta ttagcatttt ttatacttgt taggattgta gtggaatgag acattgatac 1500
gtgcattaca agaccaaaaa aaaaaaaaaa aaaaa 1535

## Description

Pollen grains originating from weeds, in particular those belonging to the very extensive plant family *Compositae* or *Asteraceae,* are common allergen sources throughout the world. The three most important wind-pollinated genera in this family are *Ambrosia* (ragweed), *Parthenium* (feverfew), and *Artemisia vulgaris* (mugwort). In late summer and autumn, pollen of mugwort is one of the main causes of allergic reactions in central Europe and parts of Asia (J. Charpin, R. Surinyach, and A. W. Frankland (1974) Atlas of European Allergenic Pollens (Paris: Sandoz)). Approximately 10% of patients suffering from pollinosis are sensitized by mugwort pollen. Ragweed pollen represents the major source of allergenic proteins in the United States and Canada, with a prevalence of about 50% in atopic individuals (L. P. Boulet, H. Turcotte, C. Laprise, C. Lavertu, P. M. Bedard, A. Lavoie, and J. Hebert (1997) Comparative degree and type of sensitization to common indoor and outdoor allergens in subjects with allergic rhinitis and/or asthma. Clin Exp Allergy 27, 52). In Europe, ragweed allergy is not a major cause of pollinosis, but it is rapidly increasing. Ragweed was first introduced into Europe at the beginning of last century and was limited to Hungary. During the Second World War it was introduced in France, thereafter gradually expanded and is now abundant in the Rhone valley (France), northern Italy, eastern parts of Austria, Hungary and Bulgaria (G. D'Amato, F. T.

Spieksma, G. Liccardi, S. Jager, M. Russo, K. Kontou-Fili, H. Nikkels, B. Wuthrich, and S. Bonini (1998) Pollen-related allergy in Europe. Allergy 53, 567).

Nowadays, it is widely accepted that recombinant allergens represent promising tools for diagnosis and therapy of Type I allergy. The value of these molecules for diagnosis has been evaluated in detail and a panel of recombinant allergens is already available for routine diagnosis of certain inhalant allergies, including tree (birch)- and grass- pollen allergies (R. Valenta et al. (1992) Int Arch Allergy Immunol 97, 287; O. Scheiner, and D. Kraft (1995) Allergy 50, 384; D. Kraft et al. (1998) Allergy 53, 62; D. Kraft et al. (1999) Int Arch Allergy Immunol 118, 171) but not for weed pollen-allergy. Two major allergenic compounds of ragweed pollen known as antigens E (Amb a 1) and K (Amb a 2) were characterized and shown to be acidic proteins of about 38,000 Da (T. P. King (1980) Allergy 35, 187). cDNA cloning of Amb a 1 and Amb a 2 revealed that these allergens are highly homologous to each other and belong to the family of pectate lyase proteins (T. Rafnar et al. (1991) J Biol Chem 266, 1229; B. L. Rogers et al. (1991) J Immunol 147, 2547). Presently, no information about the complete structure and no molecular cloning data of any mugwort pollen allergen have been published. The only exception is a mugwort pollen allergen designated Art v 1 which is disclosed in WO99/49045. Art v 1 is not related to Amb a 1 or to any other characterized ragweed allergen. Several reports demonstrated that ragweed and mugwort pollen share common allergenic structures, i.e. antigens recognized by cross-reactive IgE antibodies in different allergen sources (C. Fernandez et al. (1993) J Allergy Clin Immunol 92, 660; R. Hirschwehr et al. (1998) J Allergy Clin Immunol 101, 196; H. S. Park et al. (1994) J Korean Med Sci 9, 213). IgE recognition of such common antigens will trigger allergic reactions in sensitized patients after contact with different allergen sources. Therefore, the characterization of mugwort pollen allergens and identification of common allergenic structures in ragweed pollen will provide basic information for the selection and production of recombinant allergens that are sufficient for the diagnosis and therapy of weed pollen-allergy.

It is therefore an object of the present invention to identify an allergen from mugwort pollen which might trigger type I allergy.

The inventors succeeded in isolating four *bona fide* and complete cDNA clones corresponding to a 40.9 kDa mugwort *(Artemisia vulgaris)* pollen allergen. The amino acid sequence of the novel 40.9 kDa mugwort pollen allergen is shown in SEQ ID NO:1.

The invention therefore relates to a polypeptide comprising an amino acid sequence which has an identity of at least 70% to the amino acid sequence as shown in SEQ ID NO:1. Preferably the polypeptide comprises an amino acid sequence which has an identity of at least 80%, more preferably an identity of at least 90%, most preferably an identity of at least 95% to the amino acid sequence as shown in SEQ ID NO:1. In a particular embodiment, the polypeptide comprises the amino acid sequence as shown in SEQ ID NO:1. As used herein, the term polypeptide refers to proteins and/or peptides having a length of at least 7 amino acids.

The degree of identity of an amino acid sequence to SEQ ID NO:1 is determined by comparing said amino acid sequence and SEQ ID NO:1 using the program "BLAST 2 SEQUENCES (blastp)" (Tatusova et al. (1999) FEMS Microbiol Lett. 174, 247-250) with the following parameters: Matrix BLOSUM62; Open gap 11 and extension gap 1 penalties; gap x_dropoff 50; expect 10.0 word size 3; Filter: none. According to the invention the sequence comparison covers at least 100 amino acids, preferably at least 200 amino acids, more preferably at least 300 amino acids, most preferably at least 350 amino acids.

The invention further relates to a polypeptide comprising a fragment of at least 18 amino acids of the amino acid sequence as shown in SEQ ID NO:1. Such polypeptide comprises at least 18 consecutive amino acids of the sequence as shown in SEQ ID NO:1. Preferably, the length of the fragment is at least 21 amino acids, more preferably at least 25 amino acids, even more preferably at least 35 amino acids, most preferably at least 50 amino acids of the amino acid sequence as shown in SEQ ID NO:1.

Another aspect of the invention is a polypeptide consisting of a fragment of at least 7 amino acids of the amino acid sequence as shown in SEQ ID NO:1. Preferably, the length of said polypeptide is at least 10 amino acids, more preferably at least 15 amino acids, even more preferably at least 25 amino acids, most preferably at least 35 amino acids. Polypeptides consisting of at least 7 amino acids can be recognized by T cells (T cell epitopes).

Yet another aspect of the invention is a polypeptide comprising a fragment of the amino acid sequence as shown in SEQ ID NO:1 wherein said fragment is capable of binding to IgE antibodies from an individual being allergic against mugwort pollen. The term "IgE antibodies" means an antibody preparation which is obtainable by per se known processes. In susceptible humans, exposure to allergens leads to an immediate type (IgE-mediated) of allergic response, which is a two-step process:
(1) On the first exposure, allergenic proteins induce IgE synthesis by B-cells (Vercelli and Geha, 1989, J. Allergy Clin. Immunol. 9: 75-83). These specific IgE antibodies then bind to the surfaces of mast cells and basophils via high-affinity Fcε receptors (FcεRl).
(2) On subsequent exposure, allergens bind and crosslink these specific IgE antibodies leading to the release of pre-formed and newly synthesized inflammatory mediators (e.g. histamine) and chemotactic substances (e.g. platelet-activating factor).
In step (1), the production of allergen-specific IgE by B lymphocytes requires the "help" of T lymphocytes (Vercelli and Geha, 1989, J. Allergy Clin. Immunol. 9: 75-83), which are activated by linear peptide fragments of the allergen. These peptides are created by antigen-presenting cells through antigen processing and are displayed at the cell surface with molecules of the major histocompatibility complex (MHC), where they become available for recognition and binding to the T cell receptor (Schwartz, 1985, Annu Rev. Immunol. 3: 237-255; Rothbart et al., 1989, Int. Immunol. 1: 479-486). In step (2), allergen-specific IgE antibodies produced by B cells circulate and bind to FcεRI receptors on mast cells and basophils, thereby serving as the receptor for the allergen. Cross-linking of these cell surface-bound IgE antibodies by allergen represents the signal for the release of preformed and newly synthesized inflammatory mediators and chemotactic substances, leading to the typical allergic inflammatory reaction.

An individual being allergic against mugwort pollen is an individual that displays specific IgE antibodies recognizing mugwort pollen proteins and have typical allergic symptoms (IgE-mediated) when exposed to mugwort pollen. Such allergic reactions can also be triggered by exposure to homologous antigens present in other allergen sources. This phenomenon is defined as cross-reactivity. The selection of patients allergic to mugwort pollen was done according to typical case history, positive skin prick test and radioallergosorbent test "RAST" >3.5.

A polypeptide is capable of binding to an antibody if its affinity to the antibody is significantly higher than that of a reference composition which does not bind to the antibody. The binding of specific IgE antibodies to a particular polypeptide or allergen can be determined by various methods, e.g. RAST, immunoblot, ELISA, using as reference sera from healthy non-allergic individuals (according to case history, negative skin prick test, negative RAST), who do not display allergen-specific IgE antibodies.

In one embodiment of the invention the polypeptide is capable of binding to IgE antibodies from an individual being allergic against ragweed pollen. The present invention makes it possible to identify allergenic structures which are common to mugwort pollen and ragweed pollen. This information is useful for the selection and production of recombinant allergens that are sufficient for the diagnosis and therapy of weed pollen-allergy. Peptides and polypeptides containing such common allergenic structures are encompassed by the present invention.

During purification of natural Amb a 1 trypsin-like pollen protease cleaves this allergen. Thus, purification often results in the isolation of a full-length 38 kDa allergen plus two fragments of 26 and 12 kDa molecular weight, designated alpha (amino acids 187 to 396, numbering including signal peptide) and beta (amino acids 43 to 180, numbering including signal peptide), respectively. All chains are still highly reactive with polyclonal rabbit antibodies and human IgE from allergic patients. It is expected that proteolytic fragments of the natural 40.9 kDa mugwort allergen corresponding to these chains would also bind IgE antibodies from allergic patients. The first one corresponding to beta chain spans amino acids 21 to 180 and the second one corresponding to alpha chain spans amino acids 181 to 396, both numberings referring to SEQ ID NO:2. Both chains are expected to have epitopes which are also present in the Amb a 1 chains resulting in the same IgE reactivity.

According to a preferred embodiment, the polypeptides of the invention are isolated polypeptides, i.e. they are in an essentially pure form. "Essentially pure" means that by separation of the polypeptides from other compounds the polypeptides are at least 75% pure, preferably at least 90 % pure, more preferably at least 95% pure. The purity of polypeptides can be determined by the skilled person using techniques known in the field, e.g. by SDS-PAGE followed by protein staining. High Performance Liquid Chromatography (HPLC) and mass spectrometry are methods suitable for analyzing polypeptides and short peptides as well.

The polypeptides of the invention can be prepared in various ways. The polypeptides may be prepared by chemical synthesis, preferably by applying solid phase methods. Methods of chemical peptide synthesis are known to the skilled person. These methods are particularly suited for the preparation of short polypeptides having a length of 7 to 50 amino acids. Longer polypeptides may be prepared by chemically or enzymatically linking peptide fragments which have been prepared by chemical synthesis. The polypeptides of the invention may also be prepared by expression of DNA sequences in host cells. These methods are described in more detail below.

The present invention also concerns various polynucleotides. The polynucleotides may be single or double stranded DNA molecules, RNA molecules or nucleic acids which can be derived therefrom. According to a first aspect, the polynucleotides of the invention encode the amino acid sequence as shown in SEQ ID NO: 1. Due to the degeneracy of the genetic code, many different polynucleotides can be envisaged which encode the amino acid sequence as shown in SEQ ID NO:1.
According to a second aspect, the polynucleotides of the invention encode a polypeptide according to the invention as described supra.

The polynucleotides described herein can be used to identify similar sequences in any variety or type of mugwort and, thus, to identify or isolate sequences which have sufficient homology to hybridize to, for example, DNA from mugwort pollen. This can be carried out, for example, under conditions of low stringency; those sequences which have sufficient homology (generally greater than 40%) can be selected for further assessment. Alternatively, high stringency conditions can be used.

In this manner, DNA of the present invention can be used to identify, in other types of weed pollen sequences encoding peptides having amino acid sequences similar to that of the 40.9 kDa protein described herein and, thus, to identify allergens in such other types of weed pollen. Thus, the present invention includes not only the 40.9 kDa protein and other mugwort allergens encoded by the present DNA sequences, but also other weed pollen allergens encoded by DNA which hybridizes to DNA of the present invention.

Therefore, according to a third aspect, the polynucleotides of the invention comprise a nucleotide sequence which has an identity of at least 75 % to the nucleotide sequence as shown in SEQ ID NO:2. Preferably, the identity is at least 80 %, more preferably at least 90 %, most preferably at least 95%. The degree of identity of a polynucleotide sequence to SEQ ID NO:2 is determined by comparing said polynucleotide sequence and SEQ ID NO:2 using the program "BLAST 2 SEQUENCES (blastn)" (Tatusova et al. (1999) FEMS Microbiol Lett. 174, 247-250) with the following parameters: Reward for a match 1; Penalty for a mismatch -2; Open gap 5 and extension gap 2 penalties; gap x_dropoff 50; expect 10.0; word size 11; Filter: None. According to the invention the sequence comparison covers at least 300 nucleotides, preferably at least 600 nucleotides, more preferably at least 900 nucleotides, most preferably at least 1100 nucleotides.

In one embodiment the polynucleotide comprises the nucleotide sequence as shown in SEQ ID NO:2 or the nucleotide sequence as shown in SEQ ID NO:3.

The invention also covers polynucleotides which are degenerate to the polynucleotides described supra. The respective complementary strands of the polynucleotides disclosed are encompassed by the present invention.

The polynucleotides of the present invention preferably are isolated polynucleotides. The polynucleotides preferably are essentially pure, i. e. they are at least 80 % pure, more preferably at least 90 % pure, most preferably at least 95 % pure. The polynucleotides according to the invention can be prepared in various ways. They may be synthesized by chemical methods usually employed in oligonucleotide synthesis. PCR based methods can be used to synthesize the polynucleotides, in particular longer polynucleotides. Longer polynucleotides can also be prepared by chemically or enzymatically linking fragments which have been synthesized using chemical methods.

Another aspect of the invention is a plasmid or a vector comprising a polynucleotide according to the invention. The plasmids may contain regulatory sequences which facilitate replication of the plasmids or transcription and/or translation of encoded sequences. Examples of such sequences are promotors, terminator sequences, etc. The plasmids may also contain nucleotide sequences encoding amino acid sequences which facilitate the purification of encoded polypeptides upon expression in a host cell. Examples of such sequences are a 6xHis tag, a FLAG tag, and sequences encoding bacterial proteins such as GST. Purification can be achieved by affinity chromatography using antibodies directed against the respective tag sequences or bacterial sequences. Plasmids and vectors containing such tag sequences or bacterial sequences are known to the skilled person.

The invention further pertains to a cell containing a plasmid or a vector and/or a polynucleotide according to the present invention. The cells may be selected from plant cells, bacterial cells, yeast cells and other cells. Preferred are cells which allow for the expression of the genes encoded by the polynucleotides of the invention. Most preferably, the cells are E. coli cells. The plasmid, vector and/or polynucleotide according to the present invention may be introduced into the cells by techniques known per se, e.g. transformation, transfection etc. The cells can contain the nucleic acid molecules only transiently or stably integrated into their genome. In particular in the latter case, the nucleic acid molecules may be truncated or fragmented due to the process of integration into the genome. Cells containing such truncated or fragmented versions of the nucleic acid molecules are within the scope of the present invention.

The cells can be used for the expression and purification of the polypeptides of the invention. The invention therefore relates to a process for the preparation of a polypeptide according to the invention comprising the step of culturing the cells described in the present invention under conditions appropriate for the expression of the polypeptide and optionally subsequently recovering the polypeptide. The cells are preferably cultured in a liquid medium under agitation. If appropriate, gene expression is induced by an inducing agent, e.g. IPTG.

Following cultivation the cells may be opened using established methods and the polypeptide may recovered by affinity chromatography. Other known methods of protein purification can be employed.

Polypeptides of the present invention can be used, for example, as "purified" allergens. Such purified allergens are useful in the standardization of allergen extracts which are key reagents for the diagnosis and treatment of ragweed allergy. Furthermore, by using peptides based on fragments of the amino acid sequence as shown in SEQ ID NO: 1, anti-peptide antisera or monoclonal antibodies can be made using standard methods. These sera or monoclonal antibodies, directed against the 40.9 kDa protein of the invention, can be used to standardize allergen extracts.

Another aspect of the invention is an antibody which is capable of binding to a polypeptide according to the present invention. The antibody may be a polyclonal or a monoclonal antibody, monoclonal antibodies, however, are preferred. In a particular embodiment the antibody is in an essentially pure form, i.e. at least 80% pure, preferably at least 90% pure.

In the case of polyclonal antibodies, the antibody composition contains many different species of antibody molecules.

The antibody may be specific for the polypeptide comprising the amino acid sequence as shown in SEQ ID NO:1. In this embodiment, the antibody does not bind to anyone of the polypeptides Amb a l.1, Amb a l.2, Amb a l.3 and Amb a 2 from ragweed pollen.

In another embodiment, however, the antibody is capable of binding to one or several of the polypeptides Amb a l.1, Amb a l.2, Amb a l.3 and Amb a 2 from ragweed pollen.

Antibodies according to the invention can be used to isolate additional components of mugwort allergens which can be used for further definition of the characteristics of this family of allergens. Furthermore, anti-peptide sera or monoclonal antibodies directed against the polypeptides of the invention can be used to standardize and define the content of skin test reagents.

The invention further concerns a pharmaceutical composition comprising a polypeptide and/or a polynucleotide and/or an antibody according to the present invention.

The materials described herein, as well as compositions containing these materials, can be used in methods of diagnosing, treating and preventing mugwort allergy. Another aspect of the invention therefore is the use of a polypeptide and/or a polynucleotide and/or an antibody according to the present invention for the preparation of a medicament for the treatment or the prevention or the diagnosis of an allergic disorder.

The medicament is preferably administered to an individual to be desensitized.

Through use of the polypeptides of the present invention, allergen preparations of consistent, well-defined composition and biological activity can be made and administered for therapeutic purposes (e.g., to modify the allergic response of a mugwort-sensitive individual to a mugwort pollen). Such polypeptides or modified versions thereof may, for example, modify B-cell response to a mugwort allergen, T-cell response to a mugwort allergen or both responses. Purified allergens can also be used to design modified derivatives or analogues which are more useful in immunotherapy than are the unmodified naturally-occurring peptides.

High doses of allergens generally produce the best symptom relief. However, many people do not tolerate high doses of allergens because of allergic reactions to the allergens. Modification of naturally-occurring allergens can be designed in such a manner that modified polypeptides which have the same or enhanced therapeutic properties as the corresponding naturally-occurring allergen but have reduced side effects, especially reduced anaphylactic reactions, can be produced. These can be, for example, a polypeptide of the present invention or a modified analogue (e.g., a polypeptide in which the amino acid sequence has been altered to modify immunogenicity and/or reduce allergenicity or to which a component has been added for the same purpose). For example, the polypeptides can be modified using the polyethylene glycol method of A. Sehon and co-workers. Short segment deletion or amino acid substitutions of the polypeptide sequence results in a weakened (weaker or no) antibody binding. This modification leads to low IgE binding and is very useful in immunotherapy due to less side effects.

Administration of the peptides of the present invention to an individual to be desensitized can be carried out using known techniques. A peptide or combination of different peptides can be administered to an individual in a composition which includes, for example, an appropriate buffer, a carrier and/or an adjuvant. Such compositions will generally be administered by injection, oral administration, inhalation, transdermal application or rectal administration. Using the structural information now available, it is possible to design a mugwort pollen polypeptide which, when administered to a mugwort-sensitive individual in sufficient quantities, will modify the individual's allergic response to a mugwort allergen. This can be done, for example, by examining the structures of the mugwort proteins, producing peptides to be examined for their ability to influence B-cell and/or T-cell responses in mugwort-sensitive individuals and selecting appropriate epitopes recognized by the cells. Synthetic amino acid sequences which mimic those of the epitopes and which are capable of down regulating allergic response to mugwort allergen can also be used.

Polypeptides or antibodies of the present invention can also be used for detecting and diagnosing mugwort allergy. For example, by combining blood or blood products obtained from an individual to be assessed for sensitivity to mugwort allergen with an isolated allergenic peptide of mugwort pollen, under conditions appropriate for binding of components (e.g., antibodies, T cells, B cells) in the blood with the polypeptide and determining the extent to which such binding occurs.

It is now also possible to design an agent or a drug capable of blocking or inhibiting the ability of mugwort allergens to induce an allergic reaction in mugwort-sensitive individuals. Such agents could be designed, for example, in such a manner that they would bind to relevant anti-mugwort IgEs, thus preventing IgE-allergen binding and subsequent mast cell degranulation. Alternatively, such agents could bind to cellular components of the immune system, resulting in suppression or desensitization of the allergic response to mugwort allergens. A non-restrictive example of this is the use of appropriate B- and T-cell epitope peptides, or modifications thereof, based on the cDNA/polypeptide structures of the present invention to suppress the allergic response to mugwort allergens. This can be carried out by defining the structures of B- and T-cell epitope peptides which affect B- and T-cell function in in vitro studies with blood cells from mugwort-sensitive individuals.

Finally, the invention relates to kits which are useful for the diagnosis, the treatment and/or the prevention of an allergic disorder comprising a polypeptide and/or a polynucleotide and/or an antibody according to the present invention. The materials described herein, as well as compositions and kits containing these materials, can be used in methods of diagnosing, treating and preventing mugwort allergy.

The present invention is based on a recombinant DNA molecule and fragments thereof coding for a 40.9 kDa mugwort *(Artemisia vulgaris)* pollen allergen, which shows sequence homology to Amb a 1, a major allergen of ragweed *(Ambrosia artemisiifolia)* pollen, on a method for the isolation of Art v 1 molecules or fragments thereof, and on an expression vector and a host cell transformed to express the nucleic acid sequences of the invention. The present invention further relates to methods of diagnostics and therapy of weed pollen-allergic patients. Because the 40.9 kDa mugwort allergen shows sequence homology to Amb a 1, this mugwort protein might represent a cross-reactive allergen and could be used for diagnosis and therapy of not only mugwort pollen allergy, but of weed pollen-allergy in general.

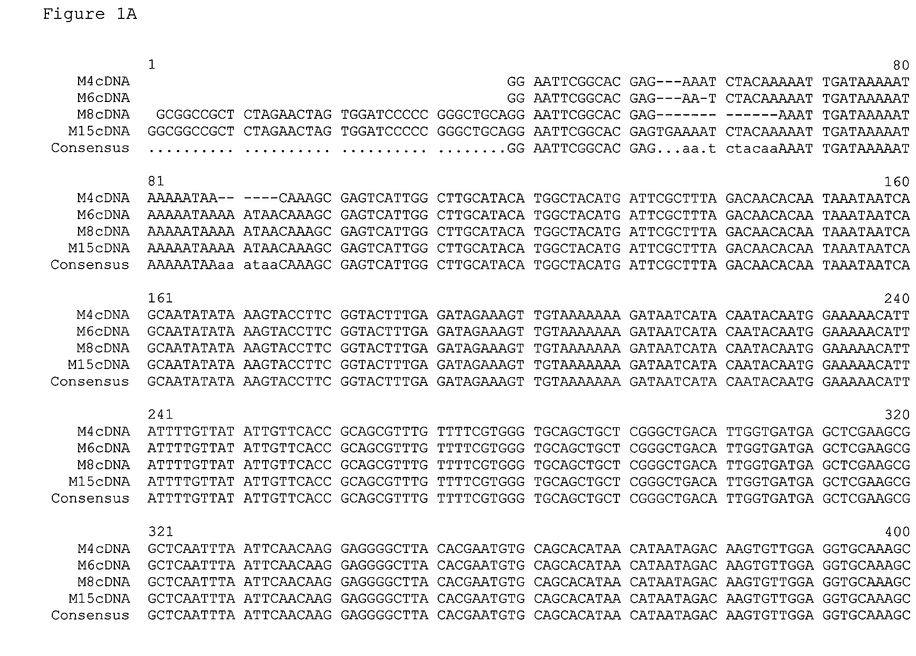

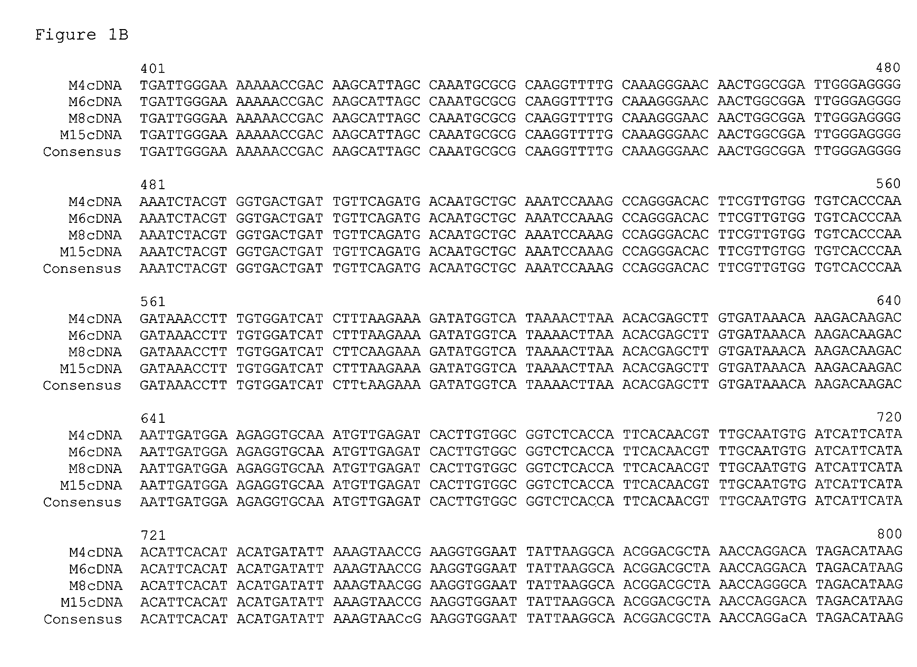

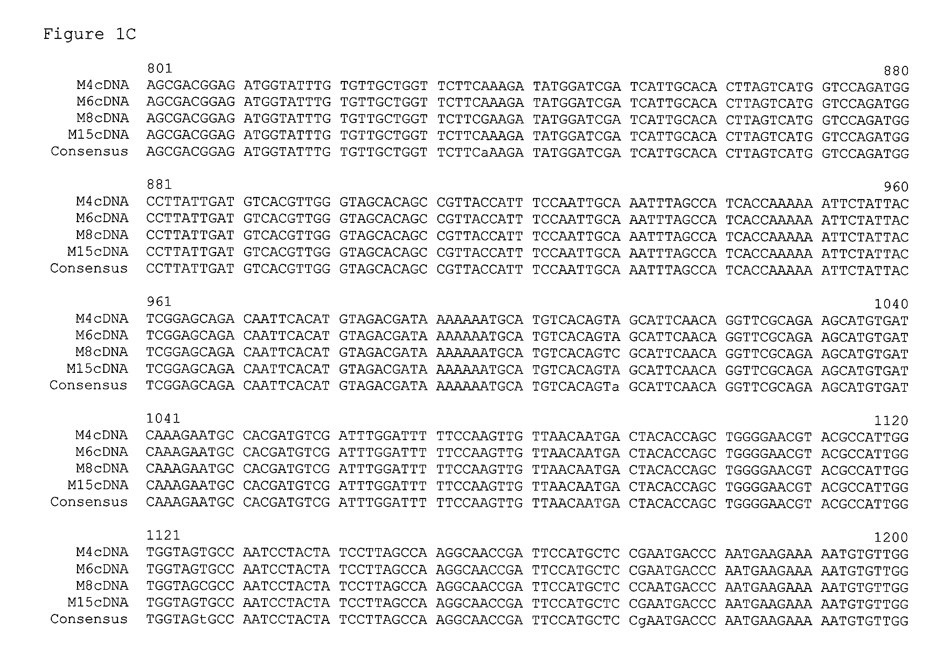

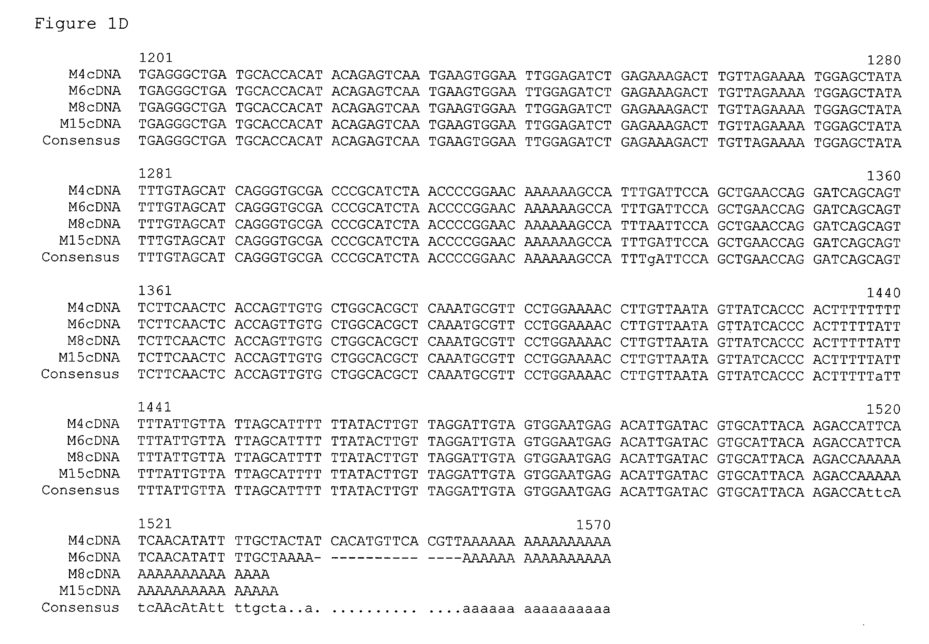

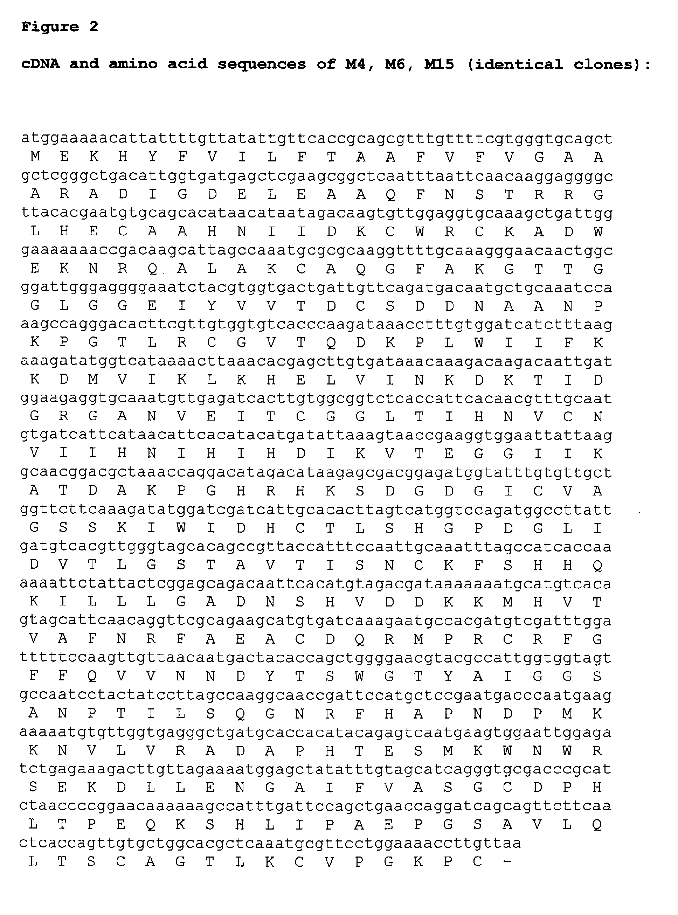

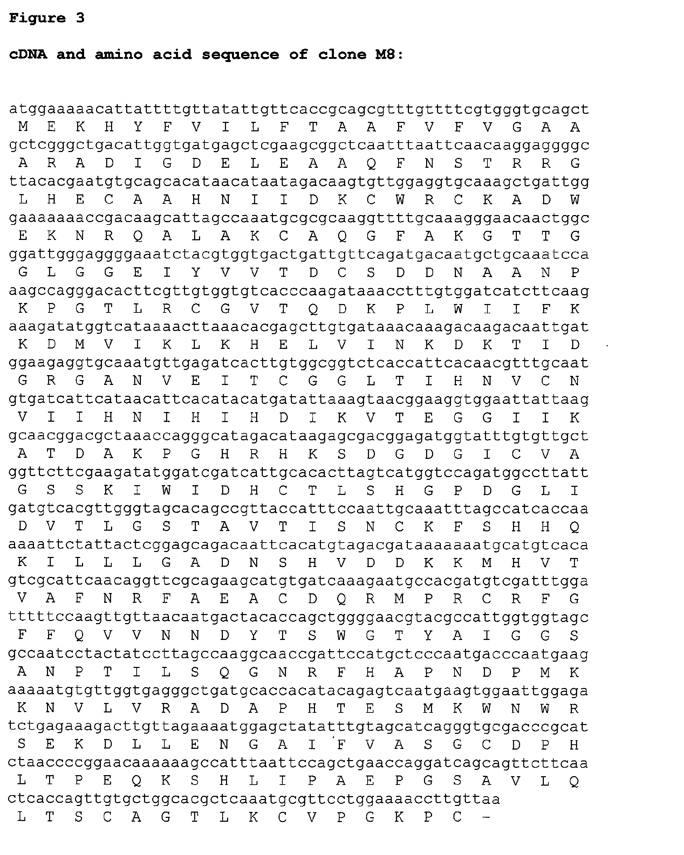

The present application describes the isolation of four *bona fide* and complete cDNA clones corresponding to a 40.9 kDa mugwort (Artemisia vulgaris) pollen allergen. The cDNA nucleotide sequences and derived amino acid sequences of the clones M4, M6, and M15 were identical (See Figures 1 and 2). Clone M8 differed from the other by 8 nucleotide substitutions, which did not lead to amino acid exchanges (See Figure 3). The clones are complete in their 5' ends because they contain the start AUG codon in a typical eukaryotic context and some sequence upstream of the start codon. The clones are complete in the 3' region because they contain 90-120 nucleotides after the stop codon followed by the polyA⁺-tail. The sequences outside the open reading frame are not shown in Figures 2 and 3. The alignments of the nucleotide sequences of clones M4, M6, M8 and M15 are shown in Figure 1A-D. The nucleotide sequence of the coding region and the deduced amino acid sequence of clones M4, M6 and M15 are shown in Figure 2. The nucleotide sequence of the coding region and the deduced amino acid sequence of clone M8 are shown in Figure 3.

Figures 1A, 1B, 1C and 1D show an alignment of the nucleotide sequences of clones M4, M6, M8 and M15 (SEQ ID NO:6, 7, 8, and 9). Capitals in the consensus sequence indicate identical nucleotides in the four clones.

Figure 2 shows the nucleotide sequence of the coding region (SEQ ID NO:2 plus stop codon) and the deduced amino acid sequence (SEQ ID NO:1) of clones M4, M6 and M15.

Figure 3 shows the nucleotide sequence of the coding region (SEQ ID NO:3 plus stop codon) and the deduced amino acid sequence (SEQ ID NO:1) of clone M8.

Figure 4 shows the structure of the multiple cloning site of plasmid "pHis-Parallel2" as used in Example 2. The amino acids encoding a 6xHis tag, a spacer region and a protease cleavage site are also indicated.

Figure 5A shows Mugwort pollen extract, purified mugwort pollen allergen and purified Amb a 1 from ragweed pollen separated by gel electrophoresis followed by Coomassie staining. Figure 5B shows an immunoblot with rabbit anti-Amb a 1 antibodies (See Examples 3 and 4).

Figure 6 shows an immunoblot with IgE antibodies (See Examples 3 and 4).

The present invention will be further illustrated by the following examples which are not intended to be limiting.

### Example 1: Immunoscreening of a Mugwort Pollen cDNA Library with Purified Rabbit anti-Amb a 1 Antibodies

The isolation of the cDNA clone coding for the 40.9 kDa mugwort pollen allergen was done in the following way:

The first step for the cDNA cloning of the 40.9 kDa allergen was the isolation of RNA from mugwort pollen, which turned out to be a very difficult procedure. Using several different standard procedures for RNA isolation, there were always pigments and other organic substances co-purifying with the RNA that inhibited enzymes used for the cDNA synthesis. However, after a series of trials it was possible to isolate mRNA sufficiently pure to construct a cDNA library, which was also done in the expression vector lambda ZAP.

Sera from rabbits immunized with Amb a 1 was kindly provided by Dr. Te Piao King, (Rockefeller University, USA). For screening the mugwort cDNA library we first purified Amb a 1-specific antibodies by affinity chromatography. For that purpose, 5 mg of Amb a 1 purified from ragweed pollen was coupled to CNBr-activated Sepharose (Pharmacia). After binding of the rabbit sera, the resin was washed and Amb a 1-specific antibodies eluted with 0.2 M glycine, pH 2.8. The antibodies were neutralized and dialyzed against PBS. With these purified rabbit anti-Amb a 1-antibodies 450.000 plaques of a mugwort pollen cDNA library constructed in lambda ZAP II phages (Stratagene, CA, USA) were screened. In total, 13 positive clones were isolated and used for single clone *in vivo excision* of pBluescript phagemid from the Uni- ZAP XR vector. Four clones (M4, M6, M8, and M15) were selected for DNA sequence analysis, which was carried out by the "primer walking" technique using 5 primers including the flanking primers T7 und T3. Both strands were sequenced twice. The aligned nucleotide sequences of the four clones M4, M6, M8, and M15 are shown in Figures 1A-1D. The cDNA sequences of clones M4, M6 and M15 were identical (Figure 2). The cDNA sequence in the coding region of M8 differed from the other 3 clones by 8 bases (Figure 3). However, all four clones lead to the same protein with a calculated molecular weight of 40.9 kDa (Figures 2 and 3).

Finally, the sequence of the 40.9 kDa protein was used for similarity searches in the Database. Significant sequence homology was found to Amb a 1, a major allergen from ragweed pollen belonging to the pectate lyase family (63.9 % identity).

The cDNA of the invention can now be obtained based on the sequence information as described herein. The cDNA can be cloned from a cDNA library using as a probe an oligonucleotide or polynucleotide which was prepared by chemical synthesis. Alternatively, the cDNA can be obtained by PCR using primers which are derived from SEQ ID NO:2. Such methods are known to the skilled person.

### Example 2: Cloning of the 40.9 kDa mugwort allergen (clone M4) into the expression plasmid pHIS- parallel-2

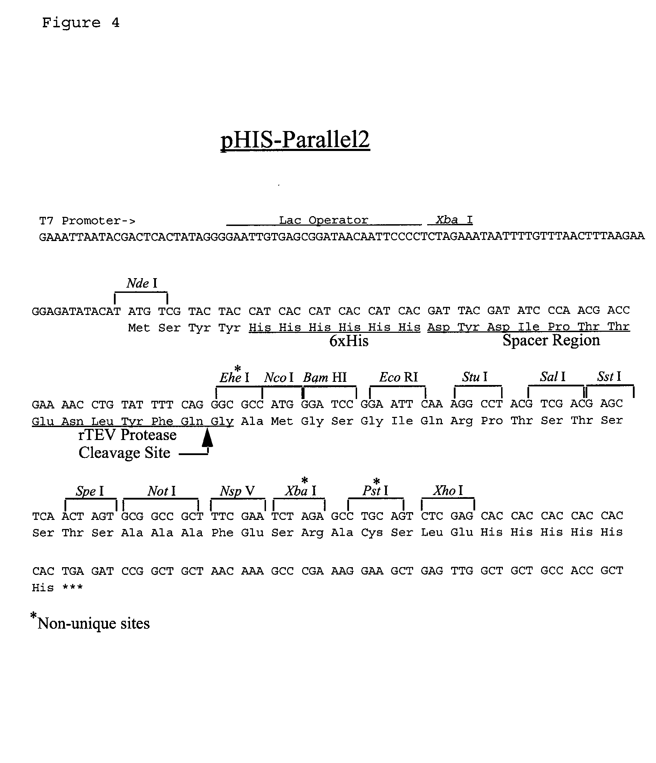

The expression plasmid containing the 40.9 kDa mugwort allergen cDNA was constructed in the vector pHis-parallel 2 (see Figure 4). For the cloning procedure two flanking cloning primers were constructed. The complete cDNA sequence was truncated at the 5' end by 60 nucleotides coding for the putative signal peptide. In this way only the mature form of the protein was produced in E. coli.

The following primers were used: Mug-Nco-fwd 5' GAG AGA GAC CAT GGC TCG GGC TGA CAT TGG TGA TGA GCT CG 3' (SEQ ID NO:4) and Mug-Xho-rev 5' GAG AGA GAC TCG AGT TAA CAA GGT TTT CCA GGA ACG CAT TTG 3' (SEQ ID NO:5) for PCR, Nco I and Xho I restriction sites were introduced at the 5' and 3' ends. PCR products were digested with Nco I and Xho I restriction enzymes and ligated to the respective sites of the vector pHis-parallel-2. The clone was analyzed by DNA sequencing.

### Example 3: Expression of the recombinant 40.9 kDa mugwort allergen in E. coli

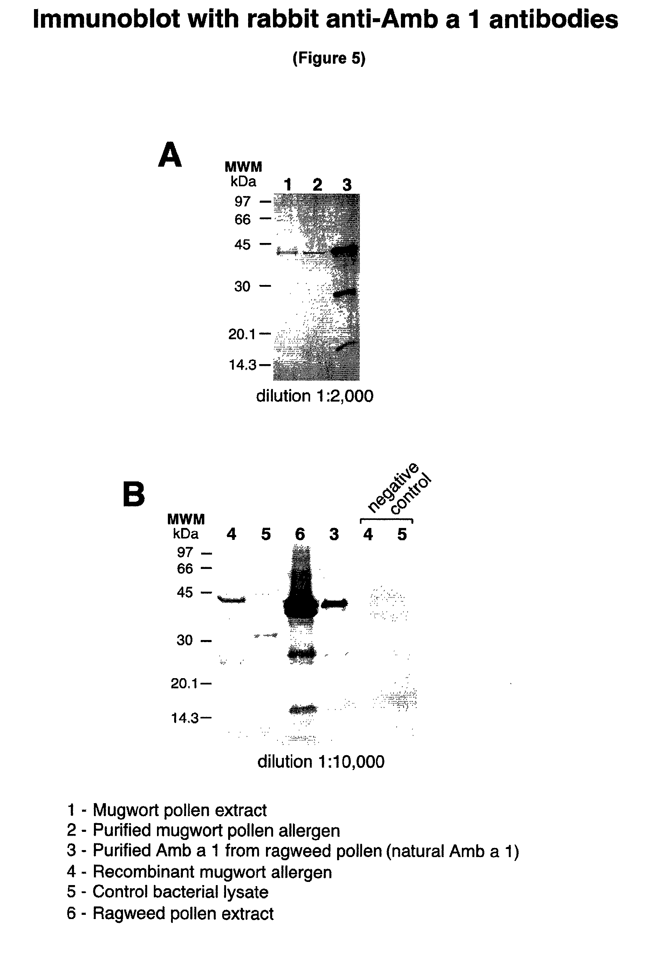

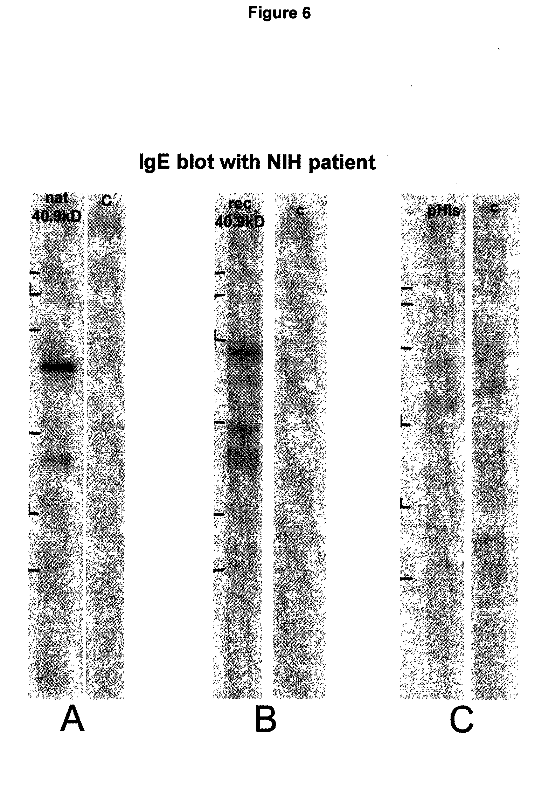

For the recombinant production of the 40.9 kDa mugwort allergen, competent E.coli strain BL21 (DE3, Stratagene, CA, USA) was transformed with the expression plasmid pHis-parallel-2-M4 and selected on LB plates containing 100 mg/l ampicillin. A single transformant colony was picked and bacteria were grown in a 25 ml overnight pre-culture with LB medium containing ampicillin to an optical density of 0.6 at 600 nm. LB-amp medium was added to a volume of 250 ml. Isopropyl-β-D-galactopyranoside (IPTG) was added to a final concentration of 1 mM and incubation continued for 7 hours at 37°C. The bacterial cells were harvested by centrifugation and pellets were resuspended in 50 mM phosphate buffer pH8 containing 300 mM NaCI and 10 mM imidazole. Cells were then disrupted by three cycles of freezing in liquid nitrogen, followed by thawing at 37°C. After centrifugation at 5.500 rpm for 25 minutes, the supernatant was removed (soluble fraction) and 6 M urea was added to the pellet. After centrifugation, the supernatant was removed (insoluble fraction), and both soluble and insoluble fractions analyzed by SDS-PAGE. The recombinant 40.9 kDa mugwort protein was recovered in the insoluble fraction, which was then used for immunoblots with anti-Amb a 1 antibodies (Figure 5B, lane 4) and with serum IgE from weed pollen-allergic patients (Figure 6, lanes 3 and 4). As control, E. coli strain BL21 transformed with pHis-parallel-2 without an insert was processed as described above and the insoluble fraction used for the immunoblot experiments (Figure 6, lanes 5 and 6).

Figure 5A, lane 1 shows the mugwort pollen extract which contains all the proteins (allergens or nonallergens) released from the pollen.
Preparation of the mugwort pollen extract:
150 mg of mugwort pollen grains (Allergon AB, Sweden) were mixed with 1 ml of water. The pollen suspension was gently agitated at room temperature for a 10 hour period. After centrifugation the pattern of proteins released from the mugwort pollen was analyzed by SDS-PAGE and Coomassie staining.

Lane 2 of Figure 5A shows purified natural 40.9 kDa mugwort pollen allergen. The purification was done via immunoaffinity chromatography method. Purified rabbit anti Amb a 1 antibodies were coupled to a cyanogen bromide acivated sepharose CL4B (Pharmacia Biotech) and used for the purification of the natural 40.9 kDa mugwort protein from a filtered 10 hours pollen extract.

Lane 3 of Figure 5A shows purified natural Amb a 1, a major allergen in the ragweed pollen. The purification was also done via immunoaffinity chromatography method. Purified rabbit anti Amb a 1 antibodies were coupled to a cyanogen bromide acivated sepharose CL4B (Pharmacia Biotech) and used for the purification of the natural Amb a 1 from a filtered 10 hours ragweed pollen extract including 10 mM protease inhibitor 1,10 phenantroline.

### Example 4: Purification of the natural 40.9 kDa allergen from mugwort pollen

The natural 40.9 kDa allergen was purified from aqueous extract of mugwort pollen by immunoaffinity chromatography. Purified rabbit anti-Amb a 1 antibodies were coupled to CNBr-activated sepharose CL4B and used for the purification of the 40.9 kDa allergen. The purified natural 40.9 kDa allergen was then tested in immunoblots with anti-Amb a 1 antibodies (Figure 5A, lane 2) and with IgE antibodies from allergic patients (Figure 6, lanes 1 and 2).

A cross-reactivity is occuring between the US patient sensitized for ragweed and the purified natural and recombinant 40.9 kDa protein in mugwort. Therefore a positive signal can be observed in Figure 6A and 6B. It is expected that many more patients show a cross-reactivity between these two allergens.

## Claims

1. A polypeptide selected from the group consisting of:
a) polypeptides comprising a fragment of at least 18 amino acids of the amino acid sequence as shown in SEQ ID NO:1;
b) polypeptides comprising an amino acid sequence which has an identity of at least 70% to the amino acid sequence as shown in SEQ ID NO:1;
c) polypeptides comprising a fragment of the amino acid sequence as shown in SEQ ID NO:1 wherein said fragment is capable of binding to IgE antibodies from an individual being allergic against mugwort pollen; and
d) polypeptides consisting of a fragment of at least 7 amino acids of the amino acid sequence as shown in SEQ ID NO:1.

2. A polypeptide according to claim 1 comprising the amino acid sequence as shown in SEQ ID NO:1.

3. A polypeptide according to claim 1 or 2 **characterized in that** it is capable of binding to IgE antibodies from an individual being allergic against ragweed pollen.

4. A polynucleotide selected from the group consisting of
a) polynucleotides encoding the amino acid sequence as shown in SEQ ID NO:1;
b) polynucleotides encoding a polypeptide as claimed in claim 1; and
c) polynucleotides comprising a nucleotide sequence which has an identity of at least 75 % to the nucleotide sequence as shown in SEQ ID NO:2;
or the complementary strand of such polynucleotide.

5. A polynucleotide according to claim 4 comprising the nucleotide sequence as shown in SEQ ID NO:2 or the nucleotide sequence as shown in SEQ ID NO:3.

6. A plasmid or a vector comprising a polynucleotide as claimed in claim 4 or 5.

7. A cell containing a plasmid or a vector as claimed in claim 6 and/or a polynucleotide as claimed in claim 4 or 5.

8. A cell according to claim 7 which is selected from the group consisting of plant cells, bacterial cells and yeast cells.

9. A process for the preparation of a polypeptide as claimed in anyone of claims 1 to 3 comprising the step of culturing cells as claimed in claim 7 or 8 under conditions appropriate for the expression of the polypeptide and optionally subsequently recovering the polypeptide.

10. A process according to claim 9 wherein the cells are opened and the polypeptide is recovered using affinity chromatography.

11. An antibody capable of binding to a polypeptide as claimed in anyone of claims 1 to 3.

12. An antibody according to claim 11 which is capable of binding to one or several of the polypeptides selected from the group consisting of Amb a 1.1, Amb a 1.2, Amb a 1.3 and Amb a 2.

13. An antibody according to claim 11 which does not bind to anyone of the polypeptides selected from the group consisting of Amb a 1.1, Amb a 1.2, Amb a 1.3 and Amb a 2.

14. A pharmaceutical composition comprising a polypeptide as claimed in anyone of claims 1 to 3 and/or a polynucleotide as claimed in claim 4 or 5 and/or an antibody as claimed in anyone of claims 11 to 13.

15. The use of a polypeptide as claimed in anyone of claims 1 to 3 or a polynucleotide as claimed in claim 4 or 5 or an antibody as claimed in anyone of claims 11 to 13 for the preparation of a medicament for the treatment or the prevention or the diagnosis of an allergic disorder.

16. A use according to claim 15 wherein the medicament is administered to an individual to be desensitized.

17. A kit useful for the diagnosis, the treatment and/or the prevention of an allergic disorder comprising a polypeptide as claimed in anyone of claims 1 to 3 and/or a polynucleotide as claimed in claim 4 or 5 and/or an antibody as claimed in anyone of claims 11 to 13.
